# EUROPEAN PATENT APPLICATION

(11) **EP 4 810 037 A1**
(43) Date of publication of application: **23.09.2026**
(21) Application number: 26166484.1
(22) Date of filing: 20.03.2026
(51) Int. Cl.: A61K 31/4245, A23L 33/00, A61P 31/00, A61P 31/04, C07D 413/12

(54) **PHARMACEUTICAL COMPOSITION FOR PREVENTING OR TREATING SEPSIS CONTAINING N-(1,3-BENZODIOXOL-5-YLMETHYL)-2-(3-PHENYL-1,2,4-OXADIAZOL-5-YL)BENZAMIDE AS AN ACTIVE INGREDIENT**

(30) Priority: 20.03.2025 KR 20250036112; 24.02.2026 KR 20260033552
(71) Applicant: Daegu Gyeongbuk Institute of Science and Technology, Daegu 42988 (KR); Research & Business Foundation Sungkyunkwan University, Gyeonggi-do 16419 (KR)
(72) Inventor: BAE, Yoe Sik, 13597 Seongnam-si (KR); JEONG, Yu Sun, 35246 Daejeon (KR); PARK, Ji Ye, 16418 Suwon-si (KR); KOO, Jaehyung, 42988 Daegu (KR); CHOI, JiWoo, 42988 Daegu (KR)
(74) Representative: Mewburn Ellis LLP

(57) **Abstract**

The present disclosure relates to a pharmaceutical composition for preventing or treating sepsis, including F177, which is an odorant receptor Olfr164 modulator, as an active ingredient. F177 was derived via establishment of a cell line that stably expresses a target odorant receptor Olfr164 and library screening in the cell line, and an activity of F177 was identified in a sepsis animal model, thereby ensuring an applicability as sepsis treatment.

## Description

### [Technical Field]

The present disclosure relates to a pharmaceutical composition for preventing or treating sepsis, including N-(1,3-benzodioxol-5-ylmethyl)-2-(3-phenyl-1,2,4-oxadiazol-5-yl)benzamide as an active ingredient.

### [Background of the Invention]

Sepsis is a severe acute infectious inflammatory disease in which an immune response is initiated by pathogens invading the body, leading to the excessive secretion of inflammatory cytokines and the induction of a systemic inflammatory response, which ultimately results in the paralysis of the body's defense ability against infectious agents due to tissue and organ damage and loss of leukocyte function. Among these, *Pseudomonas aeruginosa* is one of the representative pathogens that induce sepsis, and infection with *Pseudomonas aeruginosa* may cause pneumonia, intra-abdominal infections, postoperative wound infections, osteoarthritis, and soft tissue infections. *Pseudomonas aeruginosa* is also a representative opportunistic pathogen, and infection occurs when a patient's immunity is reduced.

Treatment for *Pseudomonas aeruginosa* known to date involves the administration of antibiotics based on the site of infection. While antibiotics such as polymyxin, levofloxacin, ciprofloxacin, and ceftazidime are commonly used, severe infections caused by *Pseudomonas aeruginosa* are difficult to treat. Furthermore, due to the increase in multidrug-resistant *Pseudomonas aeruginosa* strains resistant to many antibiotic classes (carbapenems, aminoglycosides, and fluoroquinolones), there is a growing need for control methods other than antibiotic treatment. As of 2017, multidrug-resistant *Pseudomonas aeruginosa* was reported to account for 14.7% of specimens collected from general hospitals and nursing hospitals nationwide. Due to the nature of *Pseudomonas aeruginosa*, there are limitations to antibiotic treatment because it rapidly acquires resistance even to susceptible antibiotics.

One of the most representative characteristics of *Pseudomonas aeruginosa* is its opportunistic nature, meaning it infects immunocompromised patients. Therefore, enhancing and supplementing immune function may be an effective strategy to combat *Pseudomonas aeruginosa* infection. Immune cells include innate immune cells such as neutrophils, macrophages, and dendritic cells, as well as adaptive immune cells such as B cells and T cells, however, innate immune cells play the most crucial role in the defense against *Pseudomonas aeruginosa*. Immune cells exhibit a defensive response against pathogens by recognizing various substances derived from the pathogens, and in addition to pattern-recognition receptors (PRRs) capable of recognizing pathogens, they may also recognize the external inflammatory environment and pathogens through G protein-coupled receptors (GPCRs), which primarily recognize small molecules. However, little is known about which GPCRs are activated and what functions are regulated by micrometabolites derived from pathogens.

In mammals, the odorant receptor (OR) is the largest receptor among GPCRs, accounting for more than 50% of the total. Recently, it has been reported that the physiological functions of odorant receptors are regulated not only in the nasal tissues suggested by their name, but also as they are expressed in various cells, tissues, and organs. Furthermore, as there are reports that odorant receptors may recognize micrometabolites produced by various pathogens, odorant receptors that are selectively resistant to specific bacteria may emerge as an important candidate for selecting targets for future drug development.

### [Prior-Art Documents]

### [Patent Document]

Patent Document 1. Korean Patent Registration No. 10-1616872.

### [Summary of the Invention]

### [Problem to be Solved by the Invention]

An object of the present disclosure is to provide a pharmaceutical composition for preventing or treating sepsis.

Another object of the present disclosure is to provide a health functional food composition for preventing or improving sepsis.

Another object of the present disclosure is to provide a reagent composition for modulating an odorant receptor Olfr164.

### [Means for Solving the Problem]

In order to achieve the above object, the present disclosure provides a pharmaceutical composition for preventing or treating sepsis including, as an active ingredient, a compound selected from a compound represented by Chemical Formula 1 below, a pharmaceutically acceptable salt thereof, a solvate thereof, or a stereoisomer thereof.

In addition, the present disclosure provides a health functional food composition for preventing or ameliorating sepsis including, as an active ingredient, a compound selected from a compound represented by Chemical Formula 1 below, a pharmaceutically acceptable salt thereof, a solvate thereof, or a stereoisomer thereof.

In addition, the present disclosure provides a reagent composition for modulating an odorant receptor Olfr164 including, as an active ingredient, a compound selected from a compound represented by Chemical Formula 1 below, a pharmaceutically acceptable salt thereof, a solvate thereof, or a stereoisomer thereof.

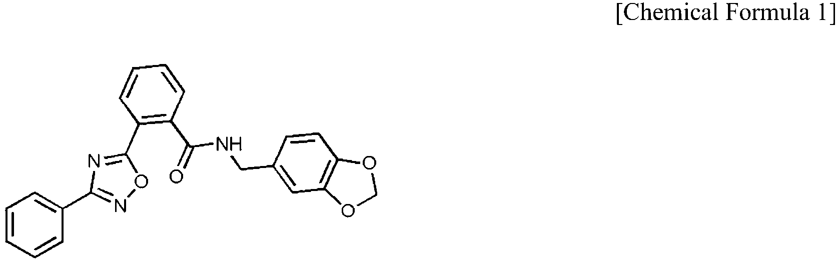

### [Effects of the Invention]

The present disclosure relates to a pharmaceutical composition for preventing or treating sepsis, including F177, which is an odorant receptor Olfr164 modulator, as an active ingredient. F177 was derived via establishment of a cell line that stably expresses a target odorant receptor Olfr164 and library screening in the cell line, and an activity of F177 was identified in a sepsis animal model, thereby ensuring an applicability as sepsis treatment.

### [Brief Description of the Drawings]

FIG. 1 shows diagrams that illustrate YFP quenching assay system.
FIG. 2 is a set of graphs showing results of measuring an intracellular cAMP level using YFP fluorescence quenching.
FIG. 3 shows diagrams illustrating the establishment of stably cAMP sensor expressing cell line and assay.
FIG. 4 shows the activity of the cAMP sensor.
FIG. 5 is a diagram showing a strategy for establishing a stable cell line using a lentiviral vector.
FIG. 6 is a set of images showing expression of a target odorant receptor Olfr164 in Hana3A and a CCY cell line.
FIG. 7 shows the results of the surface expression of target odorant receptor Olfr164 in selected cell lines.
FIG. 8 is a diagram showing a result of primary screening using a cell line that stably expresses a target odorant receptor Olfr164.
FIG. 9 is a diagram showing a result of secondary screening using a cell line that stably expresses a target odorant receptor Olfr164.
FIG. 10 is a diagram showing a result of a tertiary screening using a cell line that stably expresses a target odorant receptor Olfr164.
FIG. 11 is a diagram showing results of quaternary screening using a cell line that stably expresses a target odorant receptor Olfr164.
FIG. 12 is a set of diagrams showing results of quaternary screening of five selected compounds.
FIG. 13 is a set of graphs showing results of identifying specific responses of selected compounds to a target odorant receptor Olfr164 via a luciferase reporter assay.
FIG. 14 is a graph showing analysis of a survival rate by F177 in a sepsis animal model.
FIG. 15 is a set of diagrams showing analysis of a bactericidal effect by F177 in a sepsis animal model.
FIG. 16 is a set of graphs showing analysis of an antimicrobial activity by F177 in a sepsis animal model.
FIG. 17 is a set of diagrams showing analysis of an inhibitory effect on immune cell death by F177 in a sepsis animal model.
FIG. 18 is a set of diagrams showing analysis of an effect on inhibiting lung damage by F177 in a sepsis animal model.
FIG. 19 is a set of graphs showing analysis of modulatory effects on inflammatory cytokine production and IgM production by F177 in a sepsis animal model.
FIG. 20 is a set of graphs showing analysis of changes in major immune cells by F177 in a sepsis animal model.

### [Detailed Description]

Hereinafter, the present disclosure will be described in more detail.

The present disclosure provides a pharmaceutical composition for preventing or treating sepsis including, as an active ingredient, a compound selected from a compound represented by Chemical Formula 1 below, a pharmaceutically acceptable salt thereof, a solvate thereof, or a stereoisomer thereof.

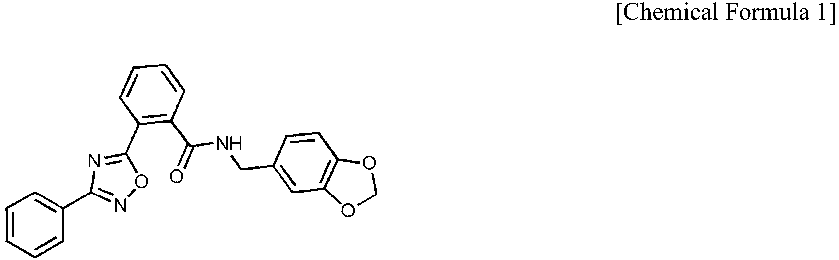

The compound may bind to an odorant receptor 164 (Olfr 164).

The sepsis may be caused by infection with *Pseudomonas aeruginosa.*

The composition may increase production of TNF-α or immunoglobulin M (IgM).

As used herein, the term "pharmaceutical composition" refers to a composition administered for the purpose of preventing or treating a specific disease, in other words, those that are administered for the treatment of sepsis for the purpose of the present disclosure.

The pharmaceutical composition according to the present disclosure may be prepared according to conventional methods in the pharmaceutical field. The pharmaceutical composition may be combined with an appropriate pharmaceutically acceptable carrier depending on the formulation, and may be manufactured by further including, if necessary, excipients, diluents, dispersants, emulsifiers, buffers, stabilizers, binders, disintegrants, and solvents. The suitable carrier is one that does not inhibit the activity and properties of the compound according to the present disclosure or a salt thereof, which may be selected depending on the dosage form and formulation.

Examples of carriers, excipients, and diluents that may be included in the pharmaceutical composition include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methylhydroxy benzoate, propylhydroxy benzoate, talc, magnesium stearate, and mineral oil.

The pharmaceutical composition may be applied in any formulation, and more specifically, it may be formulated and used as an oral formulation, an external preparation, a suppository, and a parenteral formulation of a sterile injection solution according to a conventional method, but is not limited thereto.

Among the oral formulations, the solid formulation may be in the form of tablets, pills, powders, granules, and capsules, and may be prepared by mixing at least one or more excipients, for example, starch, calcium carbonate, sucrose, lactose, sorbitol, mannitol, cellulose, and gelatin, and in addition to simple excipients, lubricants such as magnesium stearate and talc may also be included. In addition, in the case of capsule formulations, a liquid carrier such as fatty oil may be further included in addition to the aforementioned substances. Among the oral formulations, liquid formulations may include suspensions, solutions, emulsions, and syrups, and in addition to commonly used simple diluents such as water and liquid paraffin, various excipients such as wetting agents, sweeteners, fragrances, and preservatives may be included.

The parenteral formulations may include sterile aqueous solutions, non-aqueous solvents, suspensions, emulsions, lyophilized formulations, and suppositories. For non-aqueous solvents and suspensions, propylene glycol, polyethylene glycol, vegetable oils such as olive oil, and injectable esters such as ethyl oleate may be used. As for base materials of suppositories, witepsol, macrogol, tween 61, cacao butter, laurin fat, and glycerogelatin may be used. Not limited thereto, any suitable preparation known in the art may be applicable.

The pharmaceutical composition according to the present disclosure may be administered in a pharmaceutically effective amount.

As used herein, the term "pharmaceutically effective amount" refers to an amount sufficient to treat a disease at a reasonable benefit/risk ratio applicable to medical treatment without causing adverse effects.

The effective dosage level of the pharmaceutical composition may be determined differently depending on factors including the purpose of use, the patient's age, sex, weight and health condition, the type and severity of the disease, the activity of the drug, the sensitivity to the drug, the method of administration, the time of administration, the route of administration and the excretion rate, the duration of treatment, the drugs used in combination or concurrently, and other factors well known in the medical field. For example, although not constant, it may generally be administered at a dose of 0.001 to 1,000 mg/kg, preferably 0.01 to 100 mg/kg, once or several times a day. The dosage does not limit the scope of the present disclosure in any way.

The pharmaceutical composition may be administered to any animal in which sepsis may develop, and the animal may include, for example, humans and primates, as well as livestock such as cows, pigs, horses, and dogs.

The pharmaceutical composition may be administered via an appropriate route of administration depending on the form of formulations, and may be administered via various routes, such as oral or parenteral, as long as it may reach the target tissue. The method of administration is not particularly limited, and the administration may be performed by conventional methods such as oral, rectal or intravenous, intramuscular, skin application, respiratory inhalation, and intrauterine epidural or intracerebroventricular injection.

The pharmaceutical composition may be used alone for the prevention or treatment of sepsis, or may be used in combination with surgery or other drug treatments.

In addition, the present disclosure provides a health functional food composition for preventing or ameliorating sepsis including, as an active ingredient, a compound selected from a compound represented by Chemical Formula 1 below, a pharmaceutically acceptable salt thereof, a solvate thereof, or a stereoisomer thereof.

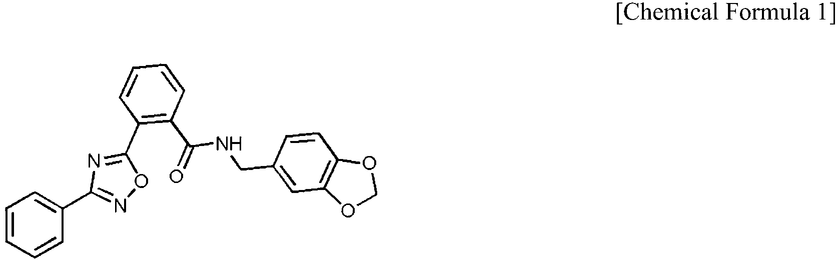

The compound may bind to an odorant receptor 164 (Olfr164).

The sepsis may be caused by infection with *Pseudomonas aeruginosa*.

As used herein, the term "health functional food" refers to a food manufactured and processed using raw materials or ingredients with functionality useful to the human body, and a food with high medical or healthcare effects, that is processed to efficiently exhibit a bioregulatory function in addition to nutritional supply, and may be used interchangeably with terms known in the art such as functional food.

The health functional food according to the present disclosure may be manufactured into a powder, granule, tablet, capsule, syrup, or beverage for the purpose of preventing or ameliorating sepsis, with no limitation on the form that the health functional food may take, and it may include all foods in the conventional sense. For example, beverages and various drinks, fruits and their processed foods (canned fruits, jams, etc.), fish, meat and their processed foods (ham, bacon, etc.), bread and noodles, cookies and snacks, and dairy products (butter, cheese, etc.) may be applicable, and all functional foods in the conventional sense may be included. In addition, it may also include food used as feed for animals.

The health functional food composition may be manufactured by further including approved food additives and other appropriate auxiliary ingredients that are commonly used in the art. Unless otherwise specified, suitability as a food additive may be determined by the standards and criteria related to corresponding items according to the general rules and general test methods of Korean Food Additives Codex approved by the Ministry of Food and Drug Safety. The items listed in the "Korean Food Additives Codex" may include, for example, chemically synthesized compounds such as ketones, glycine, potassium citrate, nicotinic acid, and cinnamic acid; natural additives such as persimmon color, licorice extracts, crystallized cellulose, kaoliang color, and guar gum; and mixed preparations such as sodium L-glutamate preparations, noodle-added alkali agents, preservative agents, and tar color agents.

The other auxiliary ingredients may additionally include, for example, flavoring agents, natural carbohydrates, sweeteners, vitamins, electrolytes, coloring agents, pectic acid, alginic acid, organic acids, protective colloid thickeners, pH adjusters, stabilizers, preservatives, glycerin, alcohol, and carbonating agents. In particular, monosaccharides such as glucose and fructose, disaccharides such as maltose and sucrose, polysaccharides such as dextrin and cyclodextrin, and sugar alcohols such as xylitol, sorbitol and erythritol may be used as the natural carbohydrates, and natural sweeteners, such as thaumatin and stevia extract, and synthetic sweeteners, such as saccharin and aspartame, may be used as the sweeteners.

The effective dosage of the compound or a salt thereof contained in the health functional food composition may be appropriately adjusted depending on the intended use, such as preventing or ameliorating sepsis.

In addition, the present disclosure provides a reagent composition for modulating an odorant receptor Olfr164, including, as an active ingredient, a compound selected from a compound represented by Chemical Formula 1 below, a pharmaceutically acceptable salt thereof, a solvate thereof, or a stereoisomer thereof.

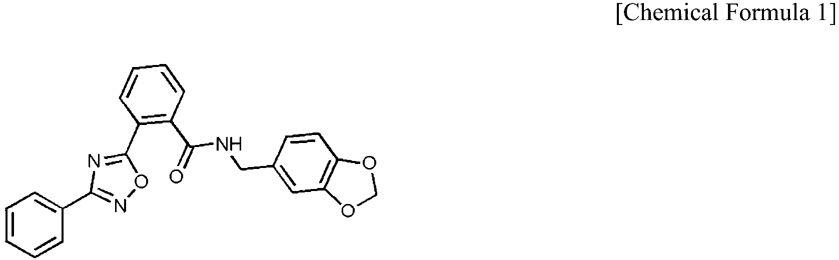

Hereinafter, to help understand the present disclosure, the present disclosure will be described in detail with reference to examples. However, the following examples are merely illustrative of the content of the present disclosure, and the scope of the present disclosure is not limited to the following examples. The examples of the present disclosure are provided to more fully explain the disclosure to those with ordinary skill in the art.

### <Example 1> Identification of stable cell lines expressing target odorant receptor Olfr164 and system establishment

### 1-1. Identification of CHO WT-CFTR YFP (CCY cells) cell line and system establishment

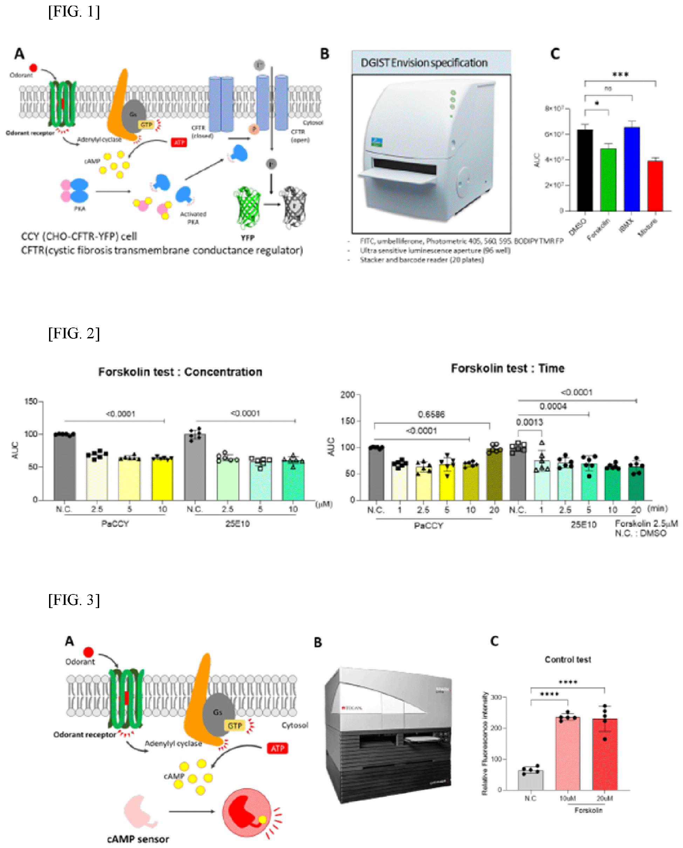

As shown in FIG. 1A, it was attempted to establish a cell line that stably expresses a target odorant receptor Olfr164 using a CHO_WT-CFTR_YFP (CCY cells) cell line. The activity of the target receptor Olfr164 was measured using a yellow fluorescent protein (YFP) quenching assay utilizing the cAMP-PKA-CFTR signaling pathway. When the compound binds to its target receptor Olfr164, the G_{S} protein is activated, leading to the adenylyl cyclase activation and an increase in the concentration of intracellular cyclic adenosine monophosphate (cAMP). Increased cAMP activates protein kinase A (PKA), which then phosphorylates and activates cystic fibrosis transmembrane conductance regulator (CFTR) expressed on the cell membrane. Iodide (I⁻) ions existing outside the cell flow into the cell through the activated CFTR channel, and the I⁻ that flowed in interacts with YFP expressed inside the cell and quenches YFP fluorescence. Here, the activity of the target receptor was evaluated by measuring the YFP fluorescence signal that decreased over time. Fluorescence activity was measured using PerkinElmer's Envision plate reader. A positive control experiment was performed to identify the responsiveness of the experimental system by treating with forskolin and IBMX, which increase intracellular cAMP levels.

### 1-2. Establishment of the method to measure the target odorant receptor Olfr164 activity using YFP quenching assay.

The regular solution and iodine solution used in the experiment were heated in a 37°C water bath before the experiment to adjust the temperature. After removing the growth media from the cells cultured in a 96-well plate, 100 µL of regular media was added to wash remaining growth media. Afterward, 99 µL of regular solution was dispensed into each well, and the 1 µL of compound to be tested was treated at each concentration. After treatment with the compound, the cells were reacted in a 37°C incubator for 10 minutes to induce intracellular cAMP production.

After the reaction was completed, 100 µL of iodide solution was added to the well and changes in the YFP fluorescence were measured immediately after iodine solution administration (Excitation 485 nm, Emission 531 nm). As a result, the YFP fluorescence was observed to decrease in proportion to the increase in intracellular cAMP levels when forskolin, an agent that elevates intracellular cAMP, was administered at varying concentrations in both cell lines PaCCY (left side of FIG. 2) and 25E10 (Right side of FIG. 2) a cell line that stable expressing target odorant receptor Olfr164.

### 1-3. Establishment of stably cAMP sensor expressing cell line and assay.

The cAMP sensor assay was prepared as a complementary approach to supplement the YFP quenching assay. As shown in FIG. 3, the assay is configured to evaluate activation of an odorant receptor using a cAMP sensor. When the receptor is activated, Gs protein-mediated signaling stimulates adenylyl cyclase, thereby increasing the intracellular concentration of cAMP. The generated cAMP binds to the cAMP sensor, resulting in elevation of an RFP fluorescence signal. Accordingly, activation of the odorant receptor is measured in an image-based system by detecting an increase in the RFP fluorescence signal.

### 1-4. Confirmation of cAMP sensor activity

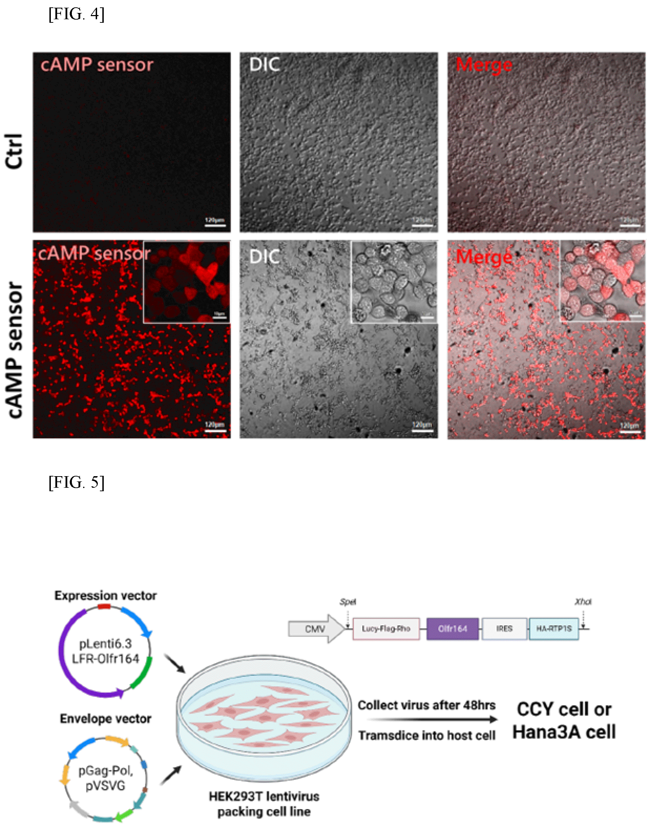

After transiently transfection of the cAMP sensor in Hana3A cell lines, a compound that increases intracellular cAMP levels was treated. As shown in FIG. 4, it was confirmed that the fluorescence signal significantly increased upon the compound treatment compared to the negative control, and the fluorescence signal showed high reproducibility and stability even during quantitative analysis.

### 1-5. Establishment of target odorant receptor clone and confirmation of its expression.

As shown in FIG. 5, the expression vector for the target odorant receptor cloned into the pLenti 6.3 vector. The cloned vector was transfected into Hana3A cells and CCY cells, respectively. Expression of the receptor was identified by western blot analysis and immunocytochemistry using an an-rho antibody that recognizes the rho tag attached to the N-terminus of the target odorant receptor.

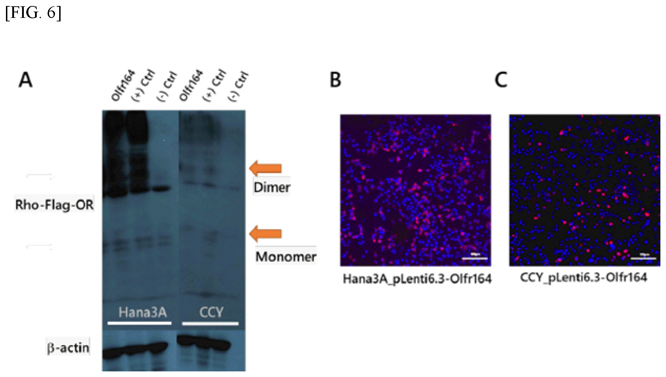

As a result, as shown in FIG. 6, the expression level of the target odorant receptor in Hana3A cells were significantly higher than in CCY cells. Additionally, as a result of western blot analysis, both monomer and dimer bands corresponding to the target odorant receptor were detected, showing that the receptor protein was normally expressed and maintained in its conformation.

### 1-6. Validation of target odorant receptor expression in selected cell lines

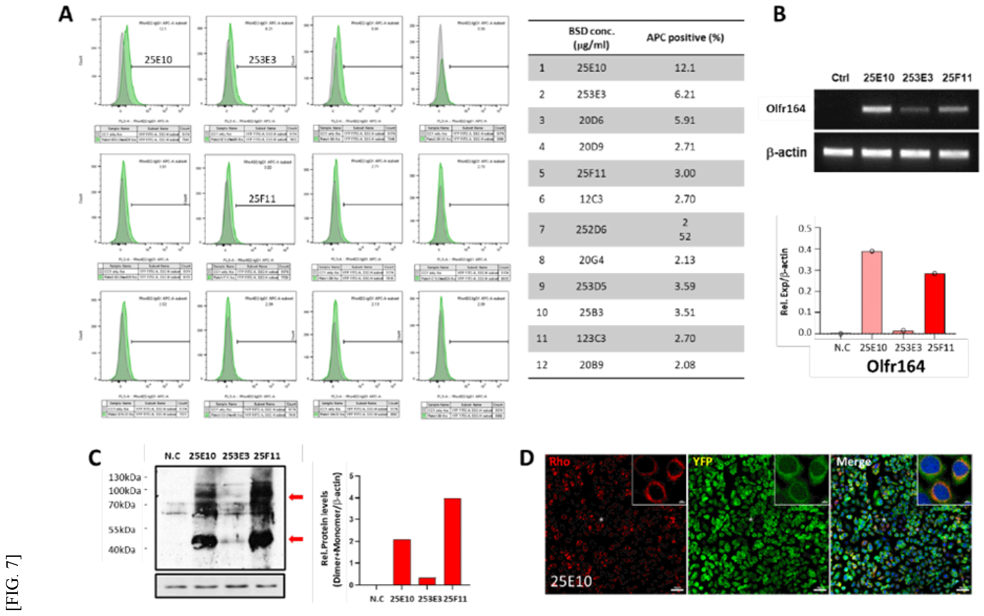

To verify the expression of target odorant receptors in the selected cell lines, fluorescence-activated cell sorting (FACS) was performed using Rho antibody as the primary antibody and allophycocyanin (APC) as the secondary antibody. As shown in FIG. 7, the surface expression of odorant receptor was identified and top 12 APC-positive cells were selected. The cell line with the highest surface expression (25E10), was selected and the expression was further analyzed with qPCR, western blot, and immune-fluorescent staining.

### <Example 2> Identification of compound libraries using stable cell lines expressing target odorant receptors

### 2-1. Primary identification of compound library (Identification and selection of effective substances)

Screening was conducted at 37.5uM (n=3) using GPCR libraries from Korea Chemical Bank (KCB). The negative control (N.C.), was the 1% dimethyl sulfoxide (DMSO) and the positive control (P.C.), was 2.5 µM forskolin, which increases intracellular cAMP level. The chemical showed higher activity than positive control was selected as primary hit.

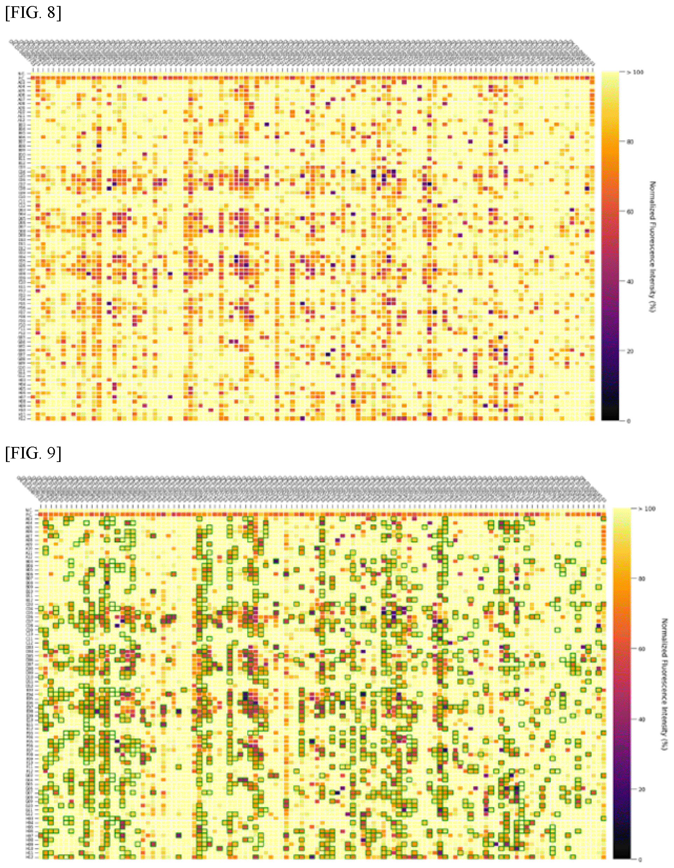

Based on these criteria, as shown in FIG. 8, 1,538 compounds were selected out of a total of 8,810 test compounds in the primary screening.

### 2-2. Secondary identification of compound library (Identification and selection of effective substances)

A total of 1,538 compounds selected through the primary screening were set as targets for the secondary screening, and these compounds are indicated by green boxes in FIG. 9. In the secondary screening, each compound was treated at the concentration of 10 µM.

Additionally, to exclude nonspecific reactions, CCY cell lines that express the other odorant receptor were used as negative controls. Based on the results of the secondary screening, compounds that showed a significant response only in the target odorant receptor-expressing cell line were selected for the tertiary screening. Based on these criteria, a total of 110 compounds were selected as candidates.

### 2-3. Tertiary identification of compound library (Identification and selection of effective substances)

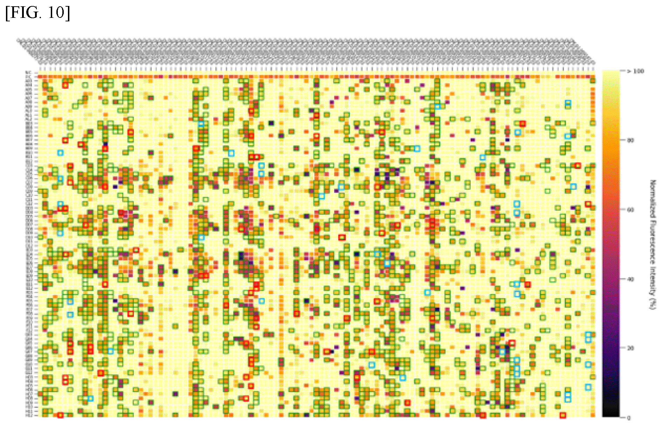

The tertiary screening was performed on a total of 110 candidate compounds, and these compounds were classified into two groups based on the results of the previous screening. The 68 compounds indicated as red boxes in FIG. 10 were compounds that were responsive in both the primary and secondary screenings, and the 42 compounds indicated as blue boxes were compounds that did not show response in the primary screening but showed significant response in the secondary screening.

The tertiary screening was performed only on cell lines expressing the target odorant receptor, and a four-step concentration gradient (0, 1, 3, 10 µM) was applied for each compound. All experiments were performed in quadruplicate (n = 4). As a result of analysis based on concentration-dependent response and reproducibility, a total of 18 compounds that stably show responses associated with target odorant receptor activity were selected as final candidates.

### 2-4. Quaternary identification of compound library (Identification and selection of effective substances)

Quaternary screening was performed on 18 candidate compounds screened through the tertiary screening. The quaternary screening was conducted using a cell line that stably expresses the target odorant receptor, and each compound was treated at a concentration gradient of 0, 0.3, 1, 3, and 10 µM. All experiments were performed in 6 repetitions (n = 6) to ensure the reproducibility and reliability of the results.

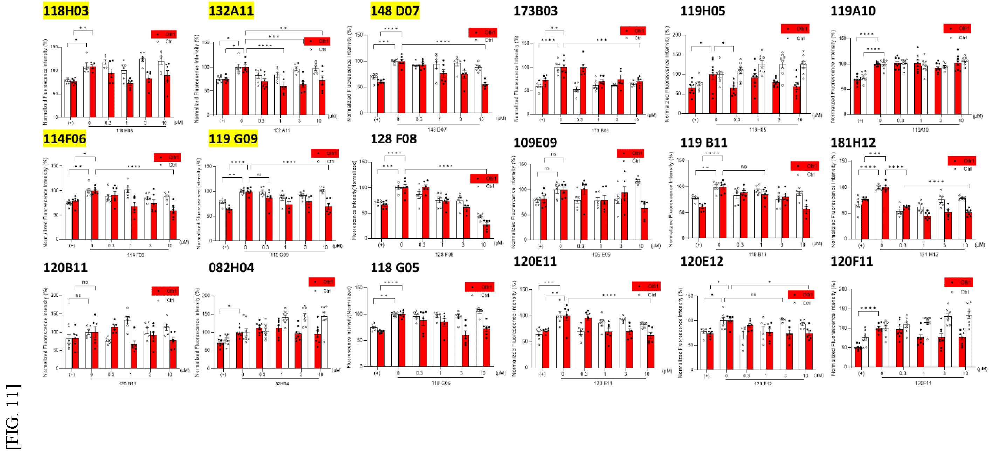

Based on the results of the quaternary screening, the final active compounds showed a significant activation of the target odorant receptor were selected, (FIG. 11), and five compounds were selected as final candidates.

### <Example 3> Reconfirmation of effective substance from quaternary identification by Luciferase assay (Identification and selection of effective substance)

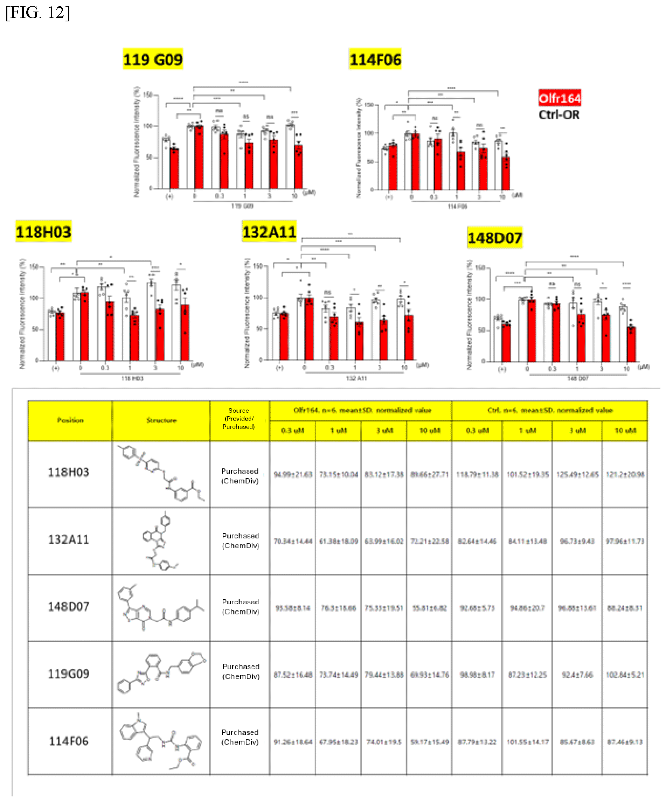

The luciferase reporter assay was conducted on the five compounds to verify whether their response with target odorant receptors is reproduced. As shown in FIG. 12, it was confirmed that the target odorant receptor-specific response was reproduced in three compounds (114F06, 132A11, 119G09) among the five selected compounds.

### <Example 4> Identification of therapeutic effects and mechanism of action of F177 (119G09), a ligand acting on a target odorant receptor, against sepsis

### 4-1. Analysis of survival rate by F177 injection in sepsis animal model

A cecal ligation and puncture (CLP) model was established to be used as a disease model mimicking human sepsis. To investigate the effectiveness in the sepsis treatment, F177 (4 mg/kg) was injected intraperitoneally a total of four times at 12-hour or 24-hour intervals starting 2 hours after the establishment of the CLP model.

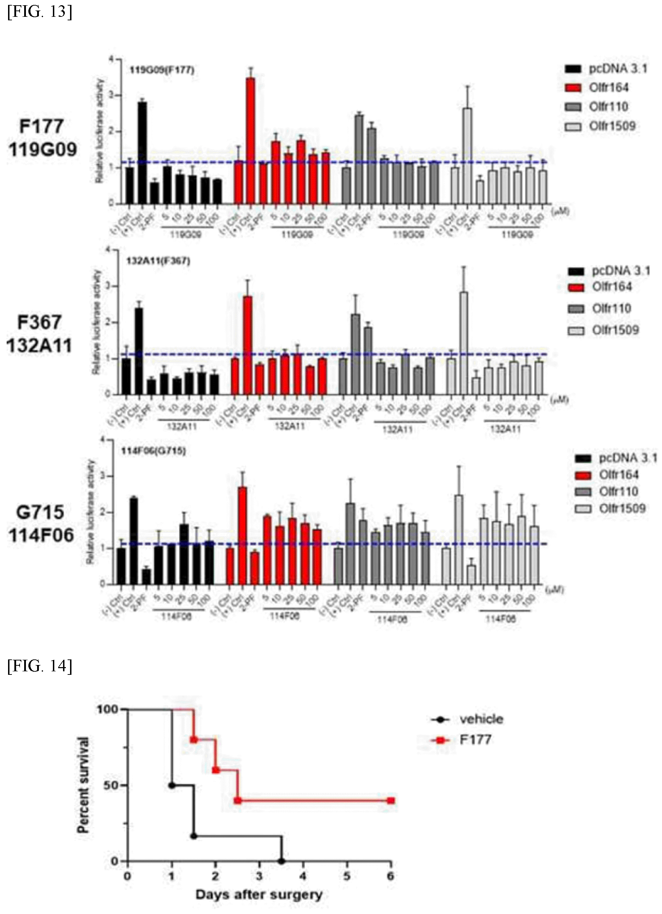

As a result, as shown in FIG. 14, all the group injected with the vehicle group (0.8% DMSO in PBS) died within 3.5 days, but 40% of the mice in the group injected with F177 at 12-hour intervals survived, showing that F177, a target odorant receptor-acting ligand, has a sepsis treatment effect.

### 4-2. Analysis of enhancement of bactericidal effect by F177 Injection in sepsis animal model

The CLP model induces a systemic inflammatory response by allowing various microorganisms present in the intestine to leak into the abdominal cavity through cecal ligation and perforation. After establishing a CLP sepsis animal model, peritoneal fluid was collected to quantitatively evaluate the bacterial load in the peritoneal cavity, and colony-forming units (CFU) were measured.

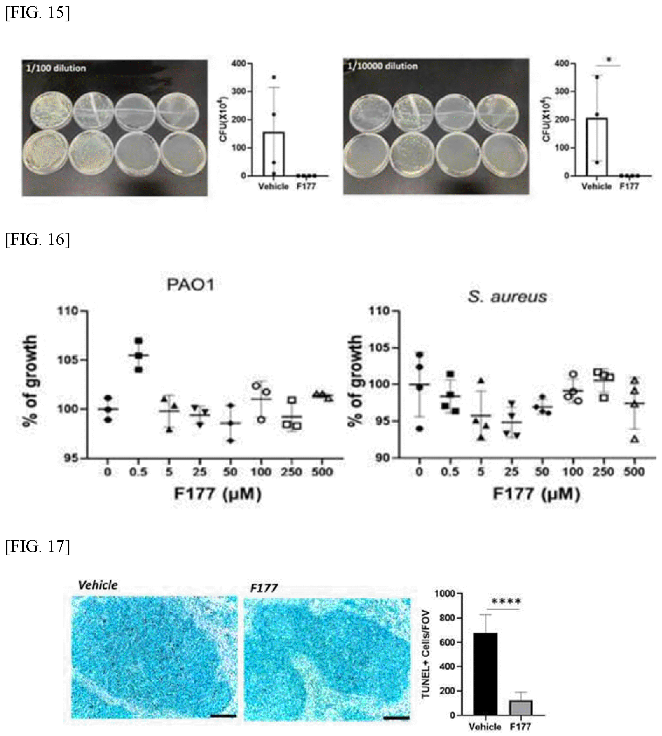

As a result, as shown in FIG. 15, the CFU of bacteria in the abdominal cavity was measured at a very high level in the CLP model group, but in the experimental group injected with F177, almost no bacterial CFU was observed in the abdominal cavity. These results indicate that intraperitoneal bacteria were effectively controlled in the CLP animal model by F177 injection, which is well consistent with the increased survival rates observed in the F177 injection group, showing that F177 brings improvement in the disease progression in the CLP-induced sepsis model through antimicrobial or infection-inhibitory effects.

By observing that the number of bacteria in the abdominal cavity decreased upon F177 injection in a CLP sepsis animal model, verification was conducted on whether this CFU reduction effect was attributed to the direct antimicrobial activity of F177. To this end, PAO1 (*Pseudomonas aeruginosa* PAO1), a representative Gram-negative bacterium, and *Staphylococcus aureus*, a Gram-positive bacterium, were cultured in vitro, and various concentrations of F177 were added to the culture medium to compare and analyze the effect on the bacterial growth.

As a result, as shown in FIG. 16, the growth curves of PAO1 and S. aureus under the condition treated with F177 did not show a significant difference from the control group, with no bacterial growth inhibitory effect observed following the F177 treatment. Through these results, it was confirmed that F177 does not possess antimicrobial activity that directly kills bacteria or inhibits their proliferation.

### 4-3. Analysis of inhibitory effect on immune cell death by F177 injection in sepsis animal model

In the CLP sepsis animal model, the decrease in the immune activity is known to be mediated by the death of immune cells in the spleen. To evaluate the degree of cell death, terminal deoxynucleotidyl transferase dUTP nick end labeling (TUNEL) analysis was performed. Spleen paraffin sections were subjected to permeabilization with a 100 mM sodium citrate solution at 70°C for 30 minutes, and then blocking was followed using normal goat serum at room temperature for 30 minutes to block non-specific binding. Subsequently, TUNEL staining was performed using the In Situ Cell Death Detection Kit. After establishing a CLP sepsis animal model, it was confirmed that terminal deoxynucleotidyl transferase dUTP nick end labeling (TUNEL)-positive immune cells significantly increased in the spleen. On the other hand, in the experimental group injected with F177, the number of TUNEL-positive immune cells was significantly reduced, as shown in FIG. 17. These results indicate that immune cell death in septic subjects was suppressed by F177 injection, indicating a possibility of an increase in the survival rate of the subjects.

### 4-4. Analysis of effect on lung injury modulation by F177 injection in sepsis animal model

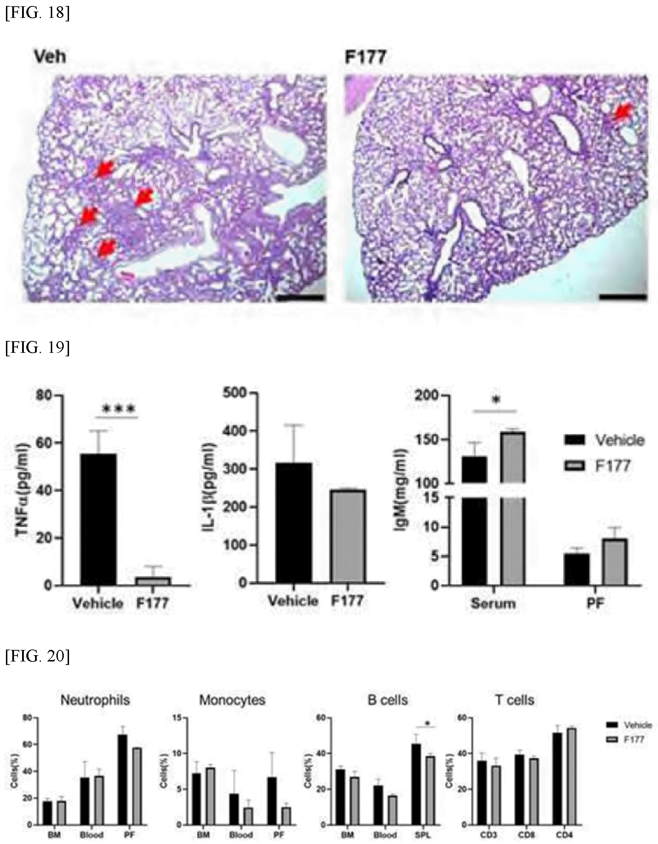

After injecting F177 a total of two times into a CLP sepsis model, the degree of tissue damage was observed via H&E staining. The lungs and spleen were removed and histologically analyzed. The excised tissue was fixed in neutral buffered formalin and then sections were prepared to a thickness of 4 µm through a paraffin molding process. The extent of tissue damage was observed using an optical microscope through hematoxylin and eosin (H&E) staining. As shown in FIG. 18, distinct lung damage was observed from a result of establishing a CLP sepsis animal model. In the F177 injection group, lung damage was observed to be alleviated, although there were differences for various parts of the lung. These results were consistent with the significant reduction in intraperitoneal CFU and enhanced effects regarding the survival rate by F177 injection in an animal model of CLP sepsis.

### 4-5. Analysis of effects of modulation of inflammatory cytokine production and immunoglobulin (IgM) production by F177 injection in sepsis animal model

The effect of modulation of inflammatory cytokine production following F177 administration in a sepsis animal model was analyzed. Mice were sacrificed at 24 hours after infection, and peritoneal lavage fluid and peripheral blood were recovered. Cytokine concentrations in the peritoneal lavage fluid and serum were measured using an enzyme-linked immunosorbent assay (ELISA). As shown in FIG. 19, when a CLP sepsis animal model was established, production of TNF-α and IL-1β in the peritoneal fluid was greatly increased, showing that the production of TNF-α was significantly reduced in the experimental group injected with F177. It was confirmed that when F177 was injected into a CLP animal model, IgM production in serum significantly increased compared to the control group.

### 4-6. Analysis of immune cell changes caused by F177 injection in sepsis animal model

Investigation was conducted on whether the increase in the mouse survival rate induced by F177 in a sepsis animal model was associated with changes in major immune cells. In a CLP sepsis animal model, the expression levels of activation markers Ly6G, CD11b, CD45, CD4, CD3e, CD19, Ly6C, CD11c, and CD8a in immune cells of the bone marrow, peripheral blood, and peritoneal cavity following F177 injection were analyzed using fluorescently labeled antibodies.

As a result, as shown in FIG. 20, no changes in neutrophils and monocytes in the bone marrow, peripheral blood, and abdominal cavity were observed following the injection of F177 in the CLP sepsis animal model, but a significant decrease in the number of B cells in the spleen was observed by injection of F177 in the CLP sepsis animal model. In the case of T cells, there was no change in CD3, CD4, and CD8 T cells by F177 injection.

As specific parts of the present disclosure have been described in detail, it will be apparent to those skilled in the art that such specific descriptions are merely preferred embodiments, and the scope of the disclosure is not limited thereby. In other words, the substantial scope of the present disclosure is defined by the appended claims and their equivalents.

## Claims

1. A pharmaceutical composition for preventing or treating sepsis comprising, as an active ingredient, a compound selected from a compound represented by Chemical Formula 1 below, a pharmaceutically acceptable salt thereof, a solvate thereof, or a stereoisomer thereof:

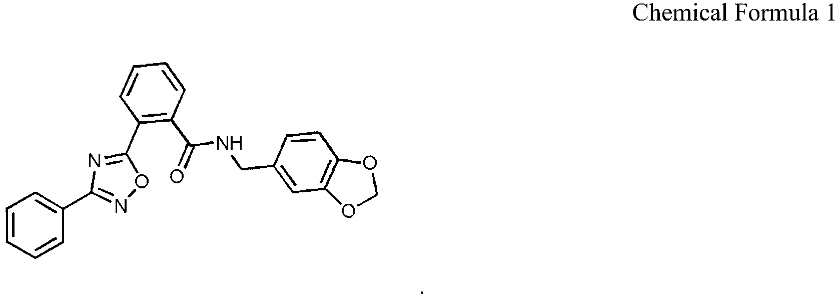

2. The pharmaceutical composition of claim 1, wherein the compound binds to an odorant receptor 164 (Olfr164).

3. The pharmaceutical composition of claim 1, wherein the sepsis is caused by infection with *Pseudomonas aeruginosa.*

4. The pharmaceutical composition of claim 1, wherein the composition increases production of TNF-α or immunoglobulin M (IgM).

5. A health functional food composition for preventing or ameliorating sepsis comprising, as an active ingredient, a compound selected from a compound represented by Chemical Formula 1 below, a pharmaceutically acceptable salt thereof, a solvate thereof, or a stereoisomer thereof:

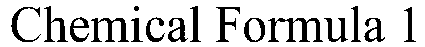

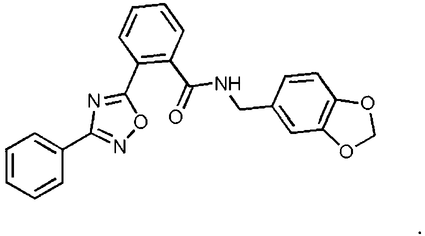

6. The health functional food composition of claim 5, wherein the compound binds to an odorant receptor 164 (Olfr164).

7. The health functional food composition of claim 5, wherein the sepsis is caused by infection with *Pseudomonas aeruginosa.*

8. A reagent composition for modulating an odorant receptor Olfr164 comprising, as an active ingredient, a compound selected from a compound represented by Chemical Formula 1 below, a pharmaceutically acceptable salt thereof, a solvate thereof, or a stereoisomer thereof:
